# EUROPEAN PATENT SPECIFICATION

(11) **EP 1 379 309 B1**
(45) Date of publication and mention of the grant of the patent: **28.06.2006**
(21) Application number: 02733901.9
(22) Date of filing: 29.03.2002
(51) Int. Cl.: A61M 25/00

(54) **MEDICAL INSTRUMENT WITH AN ATRAUMATIC END**
MEDIZINISCHES INSTRUMENT MIT ATRAUMATISCHER SPITZE
INSTRUMENT MEDICAL POURVU D'UNE EXTREMITE ATRAUMATIQUE

(30) Priority: 16.04.2001 US 283628 P
(43) Date of publication of application: 14.01.2004
(73) Proprietor: CYTYC CORPORATION, Boxborough, MA 01719 (US)
(72) Inventor: HUNG, David, Belmont, CA 94002 (US)
(74) Representative: Tombling, Adrian George
(86) International application number: PCT/US2002/009568
(87) International publication number: WO 2002/082978

(56) References cited:
- EP-A- 0 084 696
- WO-A-00/69499
- US-A- 3 996 938
- US-A- 4 344 439
- US-A- 5 700 252
- US-A- 5 788 680
- US-A- 5 860 963
- US-A- 6 017 338

## Description

The present invention relates to a medical instrument with an atraumatic end that will not cause injury as it is advanced within a patient, more specifically, the present invention relates to a medical instrument with an internal lumen and an atraumatic distal end that is free of any edges that could cut or perforate a patient while at least a portion of the instrument is being introduced and positioned in a passageway within the body of the patient.

### Background of the Invention

Many medical procedures require that a medical instrument, such as a catheter, be introduced into a passageway in the body for various reasons. These catheters are traditionally positioned and advanced into a passageway within the body. These passageways include, but are not limited to, breast ducts and other fluid carrying vessels such as blood vessels. The term "vessel" is used herein to refer to tubular and tubular-like structures within the body that carry, or are capable of carrying, fluids, cells, waste or compositions of any or all of these.

Conventional catheters have internal lumens that form passageways through which fluids or other materials are introduced and/or removed from the body. In many conventional procedures, a catheter is used to introduce fluids into a body and retrieve fluids and other compositions from within the body. One such procedure for determining if atypical cells exist in a breast duct is known as ductal lavage.

Conventional catheters include distal ends that are normally introduced into a body opening and advanced within an associated passageway using an internally positioned dilator. These known catheters have inner and outer walls and are typically cylindrical in shape. The inner and outer sidewalls each terminate at a distal edge that form a stepped shoulder that can make a sharp angle with a distal face of the catheter. These transition shoulders typically include ninety-degree angles that are capable of cutting, perforating or otherwise injuring a patient as the catheter is advanced within a body passageway. This is especially true when the distal end is passed into an area of a body passageway that has a constriction or sphincter with an opening that is smaller than the outer diameter of the catheter. At the very least, these angles and the orientation of the distal surface can cause a patient a great deal of discomfort while the catheter is being advanced within the body passageway. The discomfort is significantly increased when the catheter is passed without the use of a dilator.

US patent no. 5,700,252 discloses a nasogastric tube with a flexible tip. Patent application WO 00/69499 discloses a single operator exchange biliary catheter which has a common distal lumen. US patent no. 4,344,439 discloses a ureteral catheter with a tip of reactive reagent material. US patent no. 6,017,338 discloses an ablation catheter with a fluid cooled and perfused tip.

### Summary of the Invention

An aspect of the present invention relates to a medical instrument with an atraumatic end that will not penetrate or tear a portion of a body opening or passageway. The medical instrument according to the present invention is safer to use within a mammalian body than conventional instruments.

One aspect of the present invention relates to a catheter for inserting in a breast duct. The catheter comprises a proximal end, a distal end and a central section extending between the proximal end and the distal end. The catheter also includes an internal lumen that extends from the proximal end to the distal end through the central section. The distal end includes a rigid bulbous tip with an outer diameter that is greater than an outer diameter of the central section. The rounded surface(s) of the rigid bulbous end is safer than the end of a traditional catheter because it does not include any transition shoulders or sharp edges that could tear or otherwise perforate a passageway within a patient. As a result, the catheter will not cause pain or injury to a patient as it is advanced in a passageway within the patient.

### Brief Description of the Drawings

Fig. 1 illustrates a medical instrument according to the present invention positioned within a breast duct;
Fig. 2 is a side view of the medical instrument illustrated in Fig. 1;
Fig. 3 is a perspective view of the medical instrument illustrated in Fig. 2;
Fig. 4 is a cross-sectional view taken along the line 4-4 of Fig. 3;
Fig. 5 is an alternative embodiment of the cross-sectional view taken along the line 4-4 of Fig. 3;
Fig. 6 is an alternative embodiment of the cross-sectional view taken along the line 4-4 of Fig. 3;
Fig. 7 is an alternative embodiment of the medical instrument according to the present invention with a portion broken away;
Fig. 8 illustrates a medical instrument according to the present invention positioned within an external dilator; and
Fig. 9 is a cross sectional view of an alternative embodiment of the medical instrument according to the present invention.

### Detailed Description of the Invention

As illustrated in the figures, the present invention relates to a medical instrument 10 that is positioned within a body passageway 1 during a medical procedure. Medical instrument 10 can include a shunt, a stent, a catheter, or any other instrument used to introduce fluids into the body and/or remove fluids from within the body. The instrument 10 can also include those medical instruments that are used to introduce compositions of fluids and medicaments, therapeutic or diagnostic agents into the body and/or remove compositions of bodily fluids, introduced agents, introduced fluids and/or portions of the body (including cells and cell clumps) from within the body.

For ease of explanation, the medical instrument 10 will be described as it relates to a catheter that is inserted into a breast duct 1 before or during a ductal access procedure, such as ductal lavage, in order to introduce a fluid into the breast duct 1 and remove a composition of bodily fluids and cells from within the duct 1. However, the catheter 10 according to the present invention is not limited to being used with ductal lavage procedures. Instead, as discussed above, it can be used in any type of a medical procedure that includes the insertion of a device including an open ended lumen into a body opening or passageway.

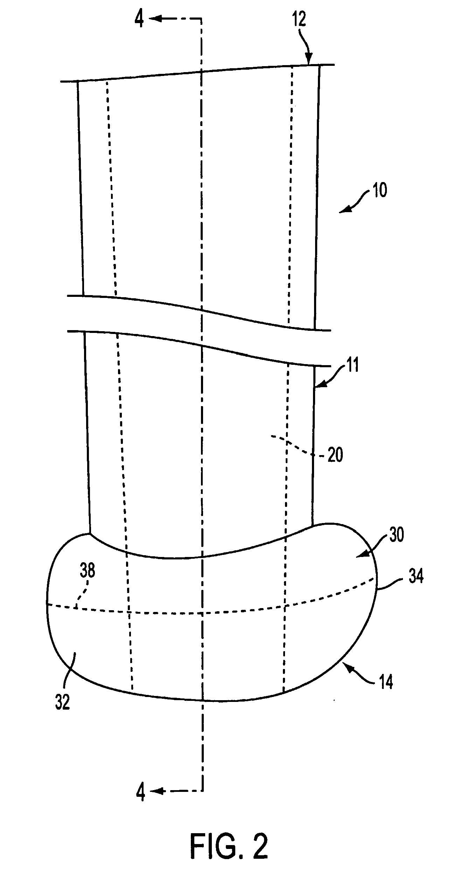

As shown in Fig. 2, the catheter 10 includes a proximal end 12, a distal end 14 and a central section 11 extending between the ends 12, 14. The proximal end 12 is similar to a proximal end of a conventional catheter. For example, the proximal end 12 has a centrally positioned opening 18 that forms a first end of an internal lumen 20. Additionally, proximal end 12 can be rigid enough that it can be grasped by a practitioner and used to steer the catheter 10 within the duct 1. Any conventional medical device that can be operatively connected to a proximal end of a conventional catheter can also be connected to the proximal end 12 of the catheter 10. For example, a fluid source could be operatively connected to end 12 in order to introduce fluids into the catheter 10 and ultimately into the body. Similarly, a collection system, with or without a vacuum source, can also be operatively connected to the proximal end 12. A system having both fluid and negative pressure sources can also be connected to the proximal end 12.

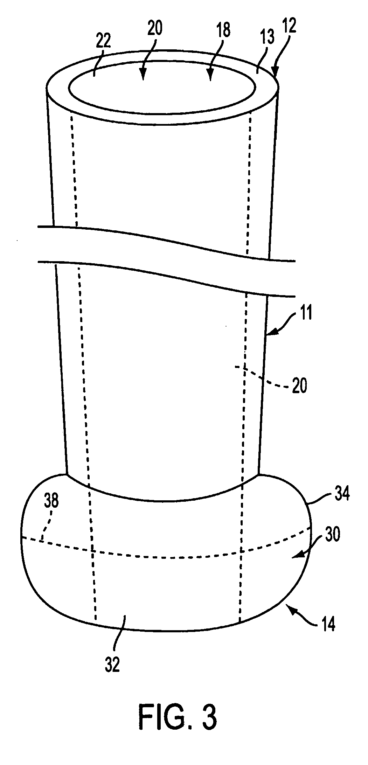

The internal lumen 20 is defined by an inner sidewall 22 that extends from an outer face 13 of the proximal end 12, along the length of the catheter 10 and to an outer, distal face 15 of the distal end 14 as shown in Fig. 3. The diameter of the inner lumen 20 is substantially the same or larger than that of conventional catheters. Similarly, the diameter of the inner lumen 20 can vary from catheter to catheter depending upon the size and purpose of the catheter 10. For example, the diameter of lumen 20 may be greater for a catheter used to deploy a balloon expandable stent within a vascular vessel than for a catheter used to deploy a balloon expandable stent within a vascular vessel than for a catheter used to recover clumps of cells from within a breast duct. No matter the purpose of the catheter, the diameter of the inner lumen 20 can be constant along the length of the catheter 10 or it can vary. In one embodiment, the diameter of the lumen 20 will taper from a point along the length of the catheter 10 to the distal end 14. The internal lumen can have a diameter of between about 0.25 mm (0.010 inch) and about 0.89 mm (0.035 inch). In one embodiment, the diameter of the internal lumen is between about 0.38 mm (0.015 inch) and 0.76 mm (0.030 inch). In another embodiment, the diameter of the internal lumen is about 0.56 mm (0.022 inch).

The outer diameter of the central section 11 of the catheter 10 is between about 0.51 mm (0.020 inch) and about 1.02 mm (0.040 inch). In another embodiment, the other diameter is between about 0.64 mm (0.025 inch) and about 0.89 mm (0.035 inch). In another embodiment, the other diameter is about 0.76 mm (0.030 inch).

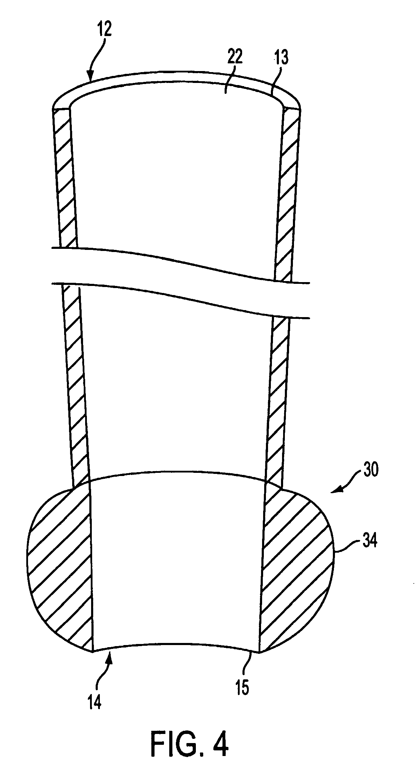

As shown in Fig. 4, the distal end 14 of the catheter 10 has a rigid, non-expandable atraumatic distal tip 30 that reduces the number of perforation or tearing injuries that are possible when a conventional catheter is advanced within a body passageway. The distal tip 30 has a substantially rounded (bulbous) shape that is void of any straight line edges resulting from the intersection of two more sections of the distal end 14 of the catheter 10. The distal, bulbous tip 30 could also have a substantially teardrop, elliptical, or other type of oval shape. Unlike the distal tips of conventional catheters, the distal tip 30 does not include an edge that may catch or otherwise snag a portion of a passageway within the patient.

As illustrated in Fig. 3, the bulbous tip 30 has an outer diameter that is greater than the outer diameter of the central section 11. As a result, the lower hemisphere 32 and the region immediately above the equator 38 of the bulbous tip 30 forms the portion of the distal end 14 that contacts the epithelial lining 40 of the breast duct 1 as the catheter 10 is advanced within the duct 1. As can be appreciated, these portions are rounded so that they are free of all sharp edges. Additionally, when the bulbous tip 30 encounters a constriction or sphincter 42 in the duct 40, the rounded sidewall 34 of the rounded lower hemisphere 32 will engage and gradually dilate the constriction 41 or sphincter 42 without perforating or cutting the lining of the duct 40. By engaging the lining of the duct and any area of reduced cross section within the duct with the rounded sidewall 34 of the lower hemisphere 32, the catheter 10 will not damage the epithelial lining 40 as it is advanced through the duct 1 without the aid of a dilator.

As discussed above, the diameter of inner lumen 20 can be constant or can taper along the length of the catheter 10. The outer diameter is substantially constant from the proximal end 12 through the central section 11. Alternatively, the outer diameter could increase from a point along central section 11 to the proximal end 14 of the bulbous tip 30. This gradual increase in the outer diameter provides additional lateral support to the bulbous tip 30.

At the distal end 14, the outside diameter of the distal tip 30 at the equator 38 is between about 0.51 mm (0.020 inch) and about 1.02 mm (0.040) inch). In another embodiment, the outer diameter of the distal tip 30 is between about 0.69 mm (0.027 inch) and about 0.94 mm (0.037 inch). In another embodiment, the outer diameter of the distal tip 30 is about 0.81 mm (0.032 inch).

The length of the catheter 10 inserted into the body during a medical procedure, including the central section 11 and the distal end 14, is between about 14.0 mm (0.55 inch) and 25.4 mm (1.0 inch). In another embodiment, this length is between about 16.5 mm (0.65 inch) and 22.9 mm (0.90 inch). In another embodiment, the length is about 20 mm (0.787 inch). In these embodiments, the length of the distal tip 30 at the distal end 14 is between about 1.40 mm (0.055 inch) and about 5.08 mm (0.20 inch) and the length of the central section 11 that is inserted into the body is between about 12.7 mm (0.50 inch) and about 20.3 mm (0.80 inch). In these embodiments, the length of the distal tip 30 can be between about 1.91 mm (0.075 inch) and 4.45 mm (0.175 inch) and the inserted length of central section 11 can be between about 14.3 mm (0.562 inch) and 19.4 mm (0.762 inch). Also, the length of the distal tip 30 can be about 3.18 mm (0.125 inch) and the inserted portion of central section 11 about 16.8 mm (0.662 inch).

The total length of the catheter 10 above the portion of point along central section 11 that is inserted into the body can be the same as the length for the portion inserted into the body. For example, the length above the portion of central section 11 inserted into the body can be about 20 mm (0.787 inch).

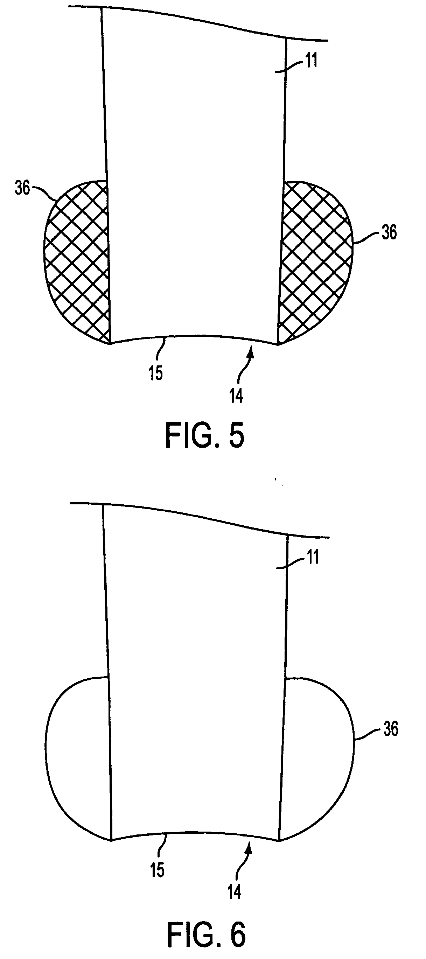

The space between the inner sidewall 22 of the lumen and the outer wall 33 of the bulbous tip 30 can be solid as shown in Fig. 4. Alternatively, it can be partially solid or hollow as shown in Figs. 5 and 6, respectively. In the partially solid embodiment, a honeycomb shaped structure 36 is located between the outer wall 33 and the inner sidewall 22, as shown in Fig. 5, to provide structural support to the bulbous tip 30. The structure 36 resists the collapsing of outer wall 33 as the catheter is advanced through the duct 40.

The catheter 10 is formed of the same rigid biocompatible materials as conventional catheters. In the hollow and partially solid embodiments, the material used for the catheter 10, especially for bulbous end 30, must be strong enough to resist collapsing in response to the forces applied to it by the ductal walls. Known materials include metals and plastics. Examples of metals used are stainless steel, nickel-titanium. One type of metal commonly used is stainless steel. Conventionally used plastics include polycarbonate, polyimides, F.E.P. Teflon, and polyurethane.

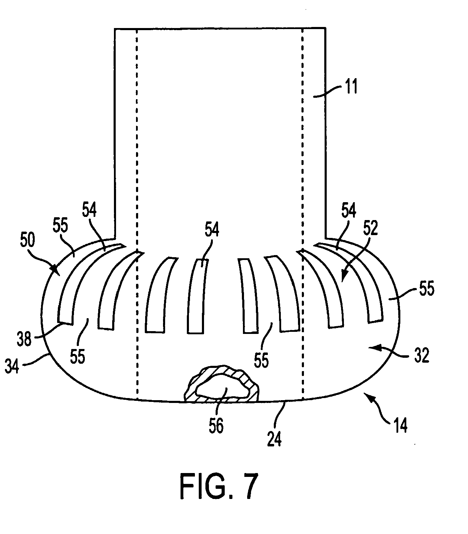

In an alternative embodiment shown in Fig. 7, an upper hemisphere 50 of the bulbous tip 30 includes a catch basket 52 for retrieving cell clumps or other materials from within the duct 40. In a preferred embodiment, circumferentially spaced rails 54 extend between the lower hemisphere 32 and the central section 11 or an upper portion of the upper hemisphere 50 to form the catch basket 52. As shown in Fig. 7, adjacent rails 54 are separated from each other by spaces 55. Each space 55 forms an opening to an internal receiving portion 56 of the basket 52 that is shown in the broken away area in Fig. 7. During the insertion and removal of the catheter 10 within the duct 40, cells that are dislodged from the epithelial lining of the duct 40 behind the bulbous tip 30 are collected in the catch basket 52 while cells in front of the bulbous tip 30 are drawn into the bulbous tip 30 and the inner lumen 20 through opening 24 in a similar manner to that disclosed in U.S. Patent Application No. 09/473,510 to Hung et al. Similarly, the basket 52 can be used to collect cells from branches of the duct that extend ninety degrees to the main branch of the duct. Typically, the ninety-degree branches are difficult to access. Therefore, the bulbous head can be passed beyond these branches so that the cell clumps flushed from these braches can be collected in basket 52 and removed from the duct.

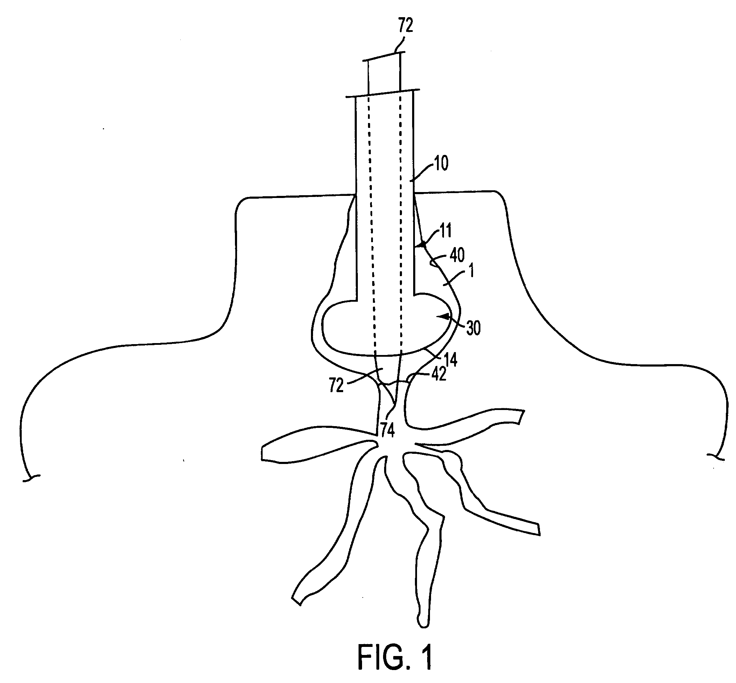

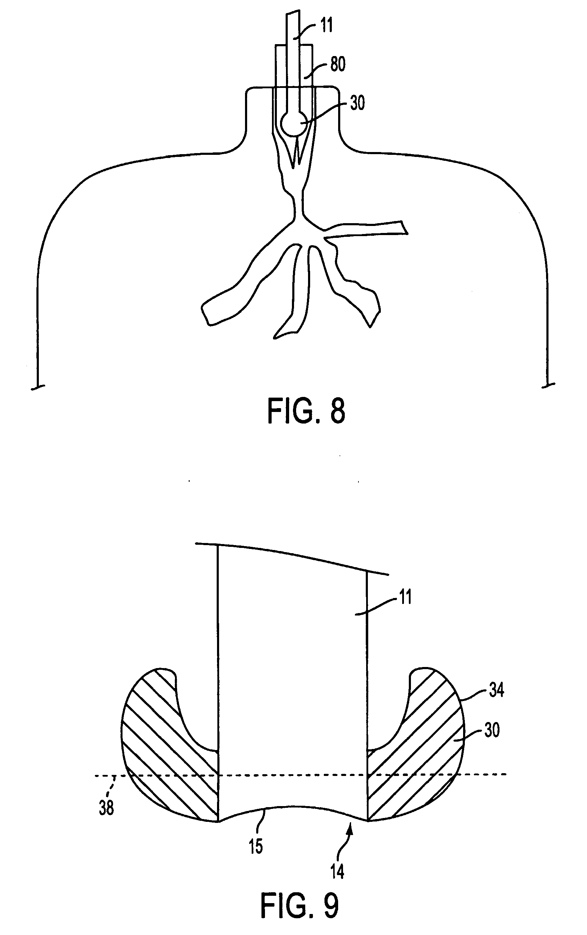

In any of the above-discussed embodiments, a conventional dilator 70 having a proximal end 72 and a distal end 74 can be used to introduce the catheter 10 into the duct I as shown in Fig. 1. As with conventional internally positioned dilators, the distal end 74 extends beyond distal end 14 of the catheter 10 in order to gradually dilate the ductal opening. Alternatively, as shown in Fig. 8, an externally positioned, expandable dilator 80 disclosed in the copending U.S. Provisional Patent Application to Hung et al. entitled "Externally Positioned Medical Dilator", can be used to dilate the ductal orifice in order to prepare it to receive the bulbous tip 30 as discussed in the above-mentioned application. As shown in Fig. 8, the bulbous tip 30 is positioned within the expandable, external dilator 80 so that the transition between the bulbous tip 30 and the dilator 80 is prevented from contacting the patient.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the scope of the appended claims. For example, as shown in Fig. 9, a portion of the bulbous end 30 above the equator 38 may have rounded flanges that form an open receiving space between the central section 11 of the catheter and the inside wall of the bulbous end below the flanges. These flanges would prevent the ends of the walls of the bulbous end from scoring or tearing the walls of the duct during the removal of the catheter.

## Claims

1. A catheter (10) for inserting in a breast duct, said catheter comprising a proximal end (12), a distal end (14), a central section (11) extending between said proximal end (12) and said distal end (14) and an internal lumen (20) extending from said proximal end (12) to said distal end (14) through said central section (11), said distal end (14) including a rigid bulbous tip (30) with an outer diameter that is greater than an outer diameter of said central section (11).

2. The catheter (10) according to claim 1 wherein said bulbous tip (30) includes at least one substantially hemispherical section (32, 34) having a substantially circular cross section.

3. The catheter (10) according to claim 1 further comprising an inner wall (22) that defines said internal lumen (20), said bulbous tip (30) includes an outer wall (33) and the space between said inner wall (22) and said outer wall (33) is solid.

4. The catheter (10) according to claim 1 wherein said bulbous end (14) is free of any sharp edges that could score or perforate a portion of the breast duct when the catheter (10) is advanced within the breast duct

5. The catheter (10) according to claim 1 wherein said bulbous tip (30) includes first (32) and second (34) hemispherical sections separated by an equator (38) that extends perpendicular to a longitudinal axis of the catheter (10).

6. The catheter (10) according to claim 5 wherein said first hemispherical section (32) is positioned between the second hemispherical section (34) and the distal end (14) of the catheter (10).

7. The catheter (10) according to claim 6 wherein said first hemispherical section (32) includes a basket (52) for collecting samples from within the breast duct.

8. The catheter (10) according to claim 7 wherein said basket (52) includes a plurality of elongated members (54) spaced by openings (55) that form a sample collection portion (56) of the basket.

9. The catheter (10) according to claim 1 further comprising an inner wall (22) that defines said internal lumen (20) and said bulbous tip (30) includes an outer wall (33) that is free of sharp edges.

10. The catheter (10) according to claim 9 wherein the space between the inner wall (22) and the outer wall (33) includes a plurality of spaced internal supporting members each separated from an adjacent one of the supporting members by a void.

11. The catheter (10) according to claim 9 wherein the space between the inner wall (22) and the outer wall (33) is hollow.

12. The catheter (10) according to claim 1 wherein the bulbous tip (30) includes a substantially circular cross section.

13. The catheter (10) according to claim 1 wherein the bulbous tip (30) is integrally formed with the central section (11) to form a continuous unitary structure.

14. The catheter (10) according to claim 1 wherein the bulbous tip (30) is substantially spherical.

15. The catheter (10) according to claim 1 wherein the bulbous tip (30) is free of edges that could injure the breast duct as the catheter (10) is introduced or removed from the breast duct.

## Patentansprüche

1. Katheter (10) zum Einführen in einen Brustgang, wobei der Katheter ein proximales Ende (12), ein distales Ende (14), einen Mittelabschnitt (11), welcher zwischen dem proximalen Ende (12) und dem distalen Ende (14) verläuft, und ein Innenlumen (20) aufweist, welches vom proximalen Ende (12) zum distalen Ende (14) durch den Mittelabschnitt (11) verläuft, wobei das distale Ende (14) eine starre, knollenförmige Spitze (30) mit einem Außendurchmesser beinhaltet, welcher größer als der Außendurchmesser des Mittelabschnitts (11) ist.

2. Katheter (10) nach Anspruch 1, wobei die knollenförmige Spitze (30) mindestens einen im Wesentlichen halbkugelförmigen Abschnitt (32, 34) mit einem im Wesentlichen kreisförmigen Querschnitt beinhaltet.

3. Katheter (10) nach Anspruch 1, welcher zudem eine Innenwand (22) aufweist, welche das Innenlumen (20) definiert, wobei die knollenförmige Spitze (30) eine Außenwand (33) beinhaltet und der Raum zwischen der Innenwand (22) und Außenwand (33) massiv ist.

4. Katheter (10) nach Anspruch 1, wobei das knollenförmige Ende (14) frei von jeglichen scharfen Kanten ist, welche einen Abschnitt des Brustgangs einschneiden oder perforieren könnten, wenn der Katheter (10) innerhalb des Brustgangs vorgeschoben wird.

5. Katheter (10) nach Anspruch 1, wobei die knollenförmige Spitze (30) erste (32) und zweite (34) halbkugelförmige Abschnitte beinhaltet, welche durch einen Äquator (38) getrennt sind, welcher senkrecht zu einer Längsachse des Katheters (10) verläuft.

6. Katheter (10) nach Anspruch 5, wobei der erste halbkugelförmige Abschnitt (32) zwischen dem zweiten halbkugelförmigen Abschnitt (34) und dem distalen Ende (14) des Katheters (10) positioniert ist.

7. Katheter (10) nach Anspruch 6, wobei der erste halbkugelförmige Abschnitt (32) einen Korb (52) zum Sammeln von Proben aus innerhalb des Brustgangs beinhaltet.

8. Katheter (10) nach Anspruch 7, wobei der Korb (52) eine Vielzahl von länglichen Elementen (54) beinhaltet, welche durch Öffnungen (55) beabstandet sind, welche einen Probensammelabschnitt (56) des Korbes bilden.

9. Katheter (10) nach Anspruch 1, welcher zudem eine Innenwand (22) aufweist, welche das Innenlumen (20) definiert, und wobei die knollenförmige Spitze (30) eine Außenwand (33) beinhaltet, welche frei von scharfen Kanten ist.

10. Katheter (10) nach Anspruch 9, wobei der Raum zwischen der Innenwand (22) und der Außenwand (33) eine Vielzahl von beabstandeten, inneren Trägerelementen beinhaltet, welche jeweils von einem angrenzenden Trägerelement durch einen Hohlraum getrennt sind.

11. Katheter (10) nach Anspruch 9, wobei der Raum zwischen der Innenwand (22) und Außenwand (33) hohl ist.

12. Katheter (10) nach Anspruch 1, wobei die knollenförmige Spitze (30) einen im Wesentlichen kreisförmigen Querschnitt aufweist.

13. Katheter (10) nach Anspruch 1, wobei die knollenförmige Spitze (30) mit dem Mittelabschnitt (11) einstückig gebildet ist, um eine kontinuierliche, einheitliche Struktur zu bilden.

14. Katheter (10) nach Anspruch 1, wobei die knollenförmige Spritze (30) im Wesentlichen kugelförmig ist.

15. Katheter (10) nach Anspruch 1, wobei die knollenförmige Spitze (30) frei von Kanten ist, welche den Brustgang verletzen könnten, wenn der Katheter (10) eingeführt oder aus dem Brustgang entfernt wird.

## Revendications

1. Cathéter (10) destiné à être inséré dans un conduit de la poitrine, le cathéter comprenant une extrémité proximale (12), une extrémité distale (14), un tronçon central (11) qui s'étend entre l'extrémité proximale (12) et l'extrémité distale (14), et une lumière interne (20) qui s'étend de l'extrémité proximale (12) à l'extrémité distale (14) par l'intermédiaire du tronçon central (11), l'extrémité distale (14) ayant un bout rigide (30) en forme de bulbe dont le diamètre externe est supérieur au diamètre externe du tronçon central (11).

2. Cathéter (10) selon la revendication 1, dans lequel le bout (30) en forme de bulbe comporte au moins un tronçon pratiquement hémisphérique (32, 34) ayant une section pratiquement circulaire.

3. Cathéter (10) selon la revendication 1, comprenant en outre une paroi interne (22) qui délimite la lumière interne (20), le bout (30) en forme de bulbe comporte une paroi externe (33), et l'espace compris entre la paroi interne (22) et la paroi externe (33) est plein.

4. Cathéter (10) selon la revendication 1, dans lequel l'extrémité (14) en forme de bulbe est dépourvue de tout bord net qui pourrait griffer ou perforer une partie du conduit de la poitrine lorsque le cathéter (10) avance dans le conduit de la poitrine.

5. Cathéter (10) selon la revendication 1, dans lequel le bout (30) en forme de bulbe comprend un premier (32) et un second (34) tronçon hémisphérique séparés par un équateur (38) qui s'étend perpendiculairement à l'axe longitudinal du cathéter (10).

6. Cathéter (10) selon la revendication 5, dans lequel le premier tronçon hémisphérique (32) est disposé entre le second tronçon hémisphérique (34) et l'extrémité distale (14) du cathéter (10).

7. Cathéter (10) selon la revendication 6, dans lequel le premier tronçon hémisphérique (32) contient un panier (52) destiné à collecter des échantillons de l'intérieur du conduit de la poitrine.

8. Cathéter (10) selon la revendication 7, dans lequel le panier (52) comporte plusieurs organes allongés (54) séparés par des ouvertures (55) qui forment une partie (56) collectrice d'échantillon du panier.

9. Cathéter (10) selon la revendication 1, comprenant en outre une paroi interne (22) qui délimite la lumière interne (20) et le bout (30) en forme de bulbe comprend une paroi externe (33) qui est dépourvue de bord net.

10. Cathéter (10) selon la revendication 9, dans lequel l'espace compris entre la paroi interne (22) et la paroi externe (33) comprend plusieurs organes internes espacés de support, séparés chacun par un vide d'un organe adjacent parmi les organes de support.

11. Cathéter (10) selon la revendication 9, dans lequel l'espace compris entre la paroi interne (22) et la paroi externe (33) est creux.

12. Cathéter (10) selon la revendication 1, dans lequel le bout (30) en forme de bulbe a une section sensiblement circulaire.

13. Cathéter (10) selon la revendication 1, dans lequel le bout (30) en forme de bulbe est formé solidairement avec le tronçon central (11) pour la formation d'une structure unitaire continue.

14. Cathéter (10) selon la revendication 1, dans lequel le bout (30) en forme de bulbe est pratiquement sphérique.

15. Cathéter (10) selon la revendication 1, dans lequel le bout (30) en forme de bulbe est dépourvu de bord qui pourrait blesser le conduit de la poitrine lorsque le cathéter (10) est introduit dans le conduit de la poitrine ou retiré de celui-ci.
